# EUROPEAN PATENT APPLICATION

(11) **EP 3 505 639 A1**
(43) Date of publication of application: **03.07.2019**
(21) Application number: 17382918.5
(22) Date of filing: 28.12.2017
(51) Int. Cl.: C12Q 1/64

(54) **COMPUTER IMPLEMENTED METHOD FOR GENERATING A PROBABILITY PROFILE OF SUBSURFACE HYDROCARBON**

(71) Applicant: Repsol, S.A., 28045 Madrid (ES)
(72) Inventor: Miranda López-Marín, Juan de Dios, 28935 MÓSTOLES (ES); Seoane Redondo, José Miguel, 28935 MÓSTOLES (ES); Esteban Rodríguez, Ángela, 28935 MÓSTOLES (ES); Espí Guzmán, Enrique, 28935 MÓSTOLES (ES); González Barroso, Maria Del Mar, 28935 MÓSTOLES (ES)
(74) Representative: ZBM Patents - Zea, Barlocci & Markvardsen

(57) **Abstract**

Examples of the present disclosure relate to a non-transitory machine-readable storage medium encoded with instructions executable by a processor. The storage medium comprises instructions to access microbiome data collected at different geographical locations, the microbiome data comprising functional profiling data. The storage medium also comprises instructions to relate biological functions of the accessed microbiome data with subsurface hydrocarbon productivity at the different geographical locations to identify biological function candidates indicative of subsurface hydrocarbon productivity. The storage medium further comprises instructions to establish a probability profile of subsurface hydrocarbon productivity associated to the biological function candidates, and instructions to identify biological function candidates which are not biological functions known to be indicative of subsurface hydrocarbon productivity.

## Description

### BACKGROUND

Both the quantity of digital data available and the processing power of computing systems are in constant increase and permit numerous data processing operations, for example using machine learning. Such data processing operations are taking place in information technology related fields such as social media for example. At the same time, other technological fields are still relying on long standing techniques which do not leverage the power of digital data combined with machine learning.

### SUMMARY OF THE INVENTION

The present invention relates to a non-transitory machine-readable storage medium encoded with instructions executable by a processor, the machine-readable storage medium comprising:
instructions to access microbiome data collected at different geographical locations, the microbiome data comprising functional profiling data;
instructions to relate biological functions of the accessed microbiome data with subsurface hydrocarbon productivity at the different geographical locations to identify biological function candidates indicative of subsurface hydrocarbon productivity;
instructions to establish a probability profile of subsurface hydrocarbon productivity associated to the biological function candidates;
instructions to identify biological function candidates which are not biological functions known to be indicative of subsurface hydrocarbon.
The invention also encompasses a related method and computer system.

### BRIEF DESCRIPTION OF THE DRAWINGS

Various example features will be apparent from the detailed description which follows, taken in conjunction with the accompanying drawings, wherein:
Figure 1 is a block diagram of an example computer system according to the present disclosure.
Figure 2 is a block diagram of example instructions encoded on a storage medium according to the present disclosure.
Figures 3a-c are schematic representations of examples of instructions to relate biological functions with subsurface productivity encoded on a storage medium according to the present disclosure.

### DETAILED DESCRIPTION

Hydrocarbon exploration is an expensive, high-risk activity where technology can be used in order to reduce associated costs. Considering that drilling success rate is rarely above 25% and that drilling a well onshore can have an approximate cost of 25 $M (million US dollars) and that offshore costs can raise from 100 $M up to 600 $M, we have developed a technology which helps reduce those risks.

Geochemical petroleum exploration techniques can be used to detect migrated surface hydrocarbons and their alteration products. In an example prospection campaign, hundreds of piston cores are extracted from surface sediments and sampled to identify and quantify different geochemical indicators that could suggest hydrocarbon presence underneath, as a prior step before final decision on further investment. A deep analysis of these samples can be used to predict hydrocarbon presence and reduce risks in the discovery of new oil and gas reservoirs.

As will be explained below, significant savings may be realized, and significant untapped value may be unlocked through the identification of biological functions which are found to be indicative of subsurface hydrocarbon productivity, beyond biological functions which are known to be indicative of subsurface hydrocarbon productivity.

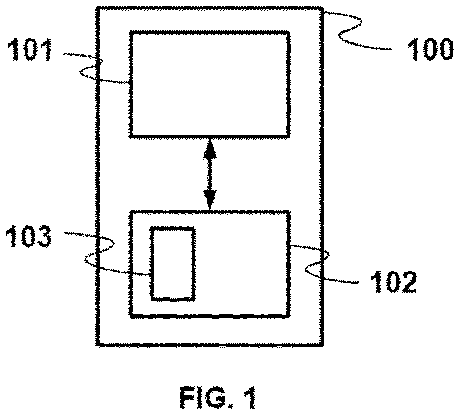

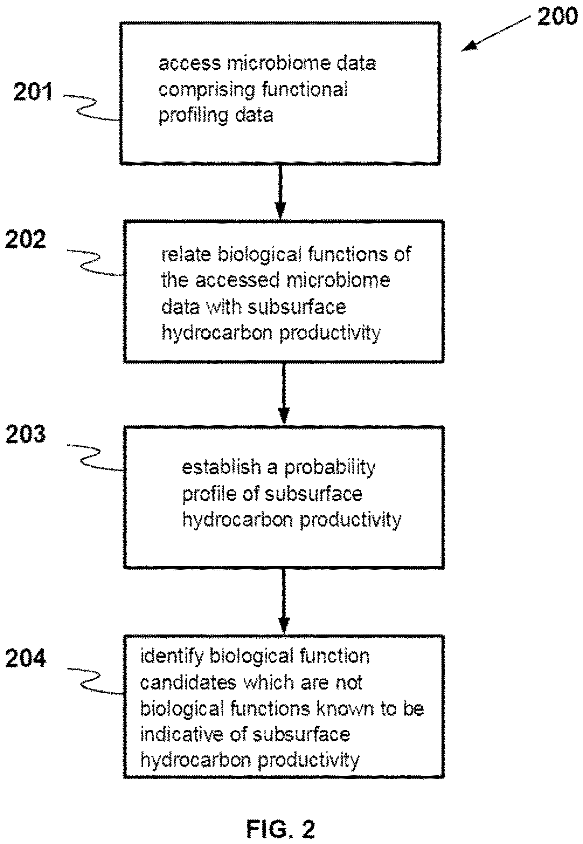

FIG. 1 illustrates in example 100 an example computer system according to the present disclosure. Computer system 100 comprises processor 101, storage medium 102 and instruction set 103. An example of instruction set 103 is represented on FIG. 2.

Processor 101 may comprise any processor, such as a central processing unit (CPU), digital signal processor (DSP), application-specific integrated circuit (ASIC), or any combination thereof. Storage medium 102 may be, for example, any electronic, magnetic, optical, or other physical storage device that stores executable instructions. Thus, machine-readable storage medium may be, for example, Random Access Memory (RAM), an Electrically-Erasable Programmable Read-Only Memory (EEPROM), a storage drive, an optical disc, and the like. Storage medium 102 is non-transitory in that it does not encompass transitory propagating signals. As used herein, the term "machine readable non-transitory storage medium" represents any device that can contain, store, retain, or maintain programs, code, scripts, information, and/or data.

In FIG. 1 storage medium 102 is disposed within computer system 100. In this situation, the instructions 103 may be installed or encoded on the storage medium 102, for example in the form of lines of computer code. Alternatively, the storage medium may be a portable, external or remote storage medium, for example, that allows a computer system to download the instructions from the portable/external/remote storage medium where they are encoded.

Referring back to FIG. 2, instructions 201-204, when executed by a processor (e.g., 101), cause computer system 100 to execute a method 200 according to the present disclosure.

In 201, microbiome data collected at different geographical locations is accessed. 201 can represent an instruction executable by a processor 101 encoded on a storage medium 102. 201 can also be comprised in a method according to the invention.

In an example microbiome data refers to the information obtained from the microorganisms present in a soil or sediment sample. Microbiome refers for example to microbial communities, such as archaea, bacteria (or bacterial soil flora), or fungi present in soil samples. Soil samples can for example be located above a hydrocarbon reservoir. The microbiome can for example contain archaea and bacteria that oxidize n-alkanes having chain lengths of 2 to 8 carbon atoms, such as methane, ethane, propane or butane. Examples of bacteria belong to genera *Acinetobacter, Actinomyces, Arthrobacter, Brevibacterium, Burkholderia, Corynebacterium, Flavobacterium, Gordonia, Methylococcus, Methylomonas, Methylobacter, Methylocyctis, Methylosinus, Methylobacterium, Mycobacterium, Nocardia, Nocardiodes, Pseudomonas, Ralstonia, Rhodococcus* or *Xanthobacter.*

In an example, the different locations are a plurality of site locations in a hydrocarbon field or exploration area. In an example, the different locations comprise one or more offshore locations. In an example, the different locations comprise one or more onshore locations, including onshore unconventional reservoirs. In an example, samples can be obtained from the first superficial centimeters up to 10 meters depth, and at other depths under sea level. In an example sampling could be done for instance by piston coring, gravity coring, or manual sampling.

The microbiome data collected in 201 comprises functional profiling data. Functional profiling data refers for example to information obtained from biological functions and metabolic pathways of the microorganisms present in a soil sample.

In 202, biological functions of the accesses microbiome data are related with subsurface hydrocarbon productivity at the different geographical locations to identify biological function candidates indicative of subsurface hydrocarbon productivity. 202 can represent an instruction executable by a processor 101 encoded on a storage medium 102. 202 can also be comprised in a method according to the invention.

A biological function can for example refer to a process performed in a microorganism present in a soil sample, which for example, allows or participates in the metabolism, growth, reproduction, movement, interaction with its environment or inter-cell communication of the microorganism.

In an example, machine learning is used to identify biological function candidates. In an example, machine learning is based at least in part on using the microbiome data as one or more training datasets; applying one or more models on the one or more training datasets to generate result data. The one or more non-transitory machine-readable storage media are provided for storing a computer database having a database schema that includes and interrelates microbiome data fields, gene feature data fields, and result data fields. The result data comprises identified biological function candidates indicative of subsurface hydrocarbon productivity.

In an example, a machine learning algorithm includes a classification algorithm. In an example, the classification algorithm corresponds to one or more of support vector machines (SVM), random forest (RF) and artificial neural networks (ANN).

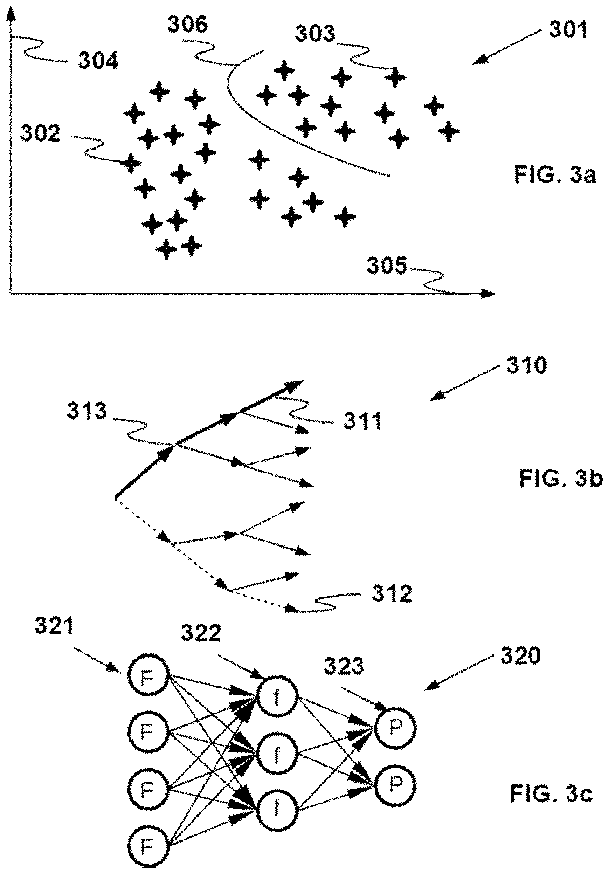

The SVM classification algorithm aims at defining an optimal hyperplane in an n-classification space, situating data points in different categories depending on which side of the hyperplane they fall. In an example, one or more hyperplanes are defined which correspond to one or more threshold of probability to indicate subsurface carbon productivity. If a specific biological function would be associated in X% of the samples in which it is present to an effective subsurface hydrocarbon productivity, and if a hyperplane P corresponds to X=X1, the specific biological function will be placed on one side of the plane if X>X1 and on the other side of the plane if X<X1. One should understand that such hyperplanes are virtual planes. The SVM classification algorithm can be trained with existing samples to locate the hyperplanes such that they follow gaps in the data. For example, if numerous functions have an X value close to an X2 and other numerous functions have an X value close to an X3 separated from X2 by a wide gap, a hyperplane can be placed between X2 and X3 separating the two groups of biological functions. Using an SVM will thereby allow classifying functions in various categories according to one or more hyperplanes, each category being associated to a range of likelihood to encounter subsurface hydrocarbon in the geographical location where the respective sample was located. A symbolic representation 301 in FIG 3a includes a group of biological functions such as biological functions 302 or 303 located on a virtual map, for example in function of presence of subsurface natural gas along axis 304 and in function of presence of petroleum along axis 305. In the representation 301, biological function 303 is more likely to indicate both the presence of natural gas and petroleum than biological function 302. Using SVM can in this example lead to defining hyperplane 306 leading to classifying the biological functions in two groups, whereby the group comprising 303 and located on one side of 306 is more likely to indicate the presence of hydrocarbons than the group comprising 302 and located on the other side of 306. In other examples, such modeling takes place along n dimensions and with high sample numbers which do not permit a schematic representation.

The RF classification algorithm can be described as an ensemble of decision trees where each tree includes branches which allow classifying samples from microbiome according to their characteristics, for example in function of the biological functions comprised in each sample. In an example, the samples are including one or more biological functions, each sample being related to an indication of subsurface hydrocarbon productivity. In an example, numerous data samples are processed by the decision tree, whereby each data sample element from the collected microbiome data follows its specific path through the decision tree. Samples having the same or similar characteristics will follow the same path within the classification tree. Running such collected microbiome data through the decision tree therefore leads in a learning phase to identifying the specific branches of the tree being linked to an indication of subsurface hydrocarbon productivity. Such learning phase may take place with a very high number of samples, leading for example to building hundreds of decision trees with many branches each, including paths that discriminate among different subsurface hydrocarbon productivity levels. In an example, RF is used to rank the biological functions. Biological functions which are related to a higher probability of subsurface hydrocarbon productivity are ranked higher than biological functions producing a lower probability of subsurface hydrocarbon productivity. Compared for example to using SVM, using RF could permit to reverse engineer the data resulting from processing the collected data on a per function basis to be in a position to classify biological functions one by one as to their likelihood or probability to be indicative of subsurface hydrocarbon productivity. A symbolic representation in FIG 3b represents a decision tree 310, whereby path 311 was identified in a learning phase as being highly indicative of subsurface carbon productivity, and whereby path 312 was identified in a learning phase as not being indicative of subsurface carbon productivity. At each intersection such as 313 a test such as for example "Is specific biological function Fa present below or above X% of all biological functions comprised in the sample" is made. This is of course a symbolic representation and RF processing of microbiome data can be significantly more complex. In an example, a sample Sa may include 100 biological functions, whereby these 100 biological functions include 40 biological functions Fa, 25 biological functions Fb, 10 biological functions Fc, and 25 other biological functions. Another sample Sb may include 200 biological functions, whereby these 200 biological functions include 100 biological functions Fa, 75 biological functions Fb, 20 biological functions Fd, and 5 other biological functions. Each sample part of the microbiome data will have such a functional profiling describing the quantity of each biological function included in the sample. In an example, RF processing builds a tree, whereby each branch of the tree represents a function profile or group of similar function profiles, whereby each branch is associated to an indication of subsurface carbon productivity. For example, sample Sb may be more indicative of subsurface carbon productivity than sample Sa. RF classification will increase in precision as additional samples are processed. This progressive process is referred to as the learning phase, whereby RF classification becomes increasingly trained, leading to discovering biological functions which are related to subsurface hydrocarbon productivity which were not known to be related to subsurface hydrocarbon productivity.

The ANN algorithm can be described as a number of artificial neurons or cells, each cell processing quantitative inputs and providing resulting outputs, the outputs being a mathematical function of the inputs, the mathematical function comprising parameters such as one or more weights, the weights being progressively adjusted as the network (i.e. the plurality of cells) is learning towards reflecting the logical structure of the data. In an example, the input can be the biological functions, the output being a related probability of subsurface hydrocarbon productivity. In a first phase, the various mathematical functions may return a first set of probability which are not in line with the actual measured probabilities, such that some probabilities will be lower than expected and some higher than expected. The ANN algorithm will then adjust the weights of the mathematical functions to raise probabilities which are lower than expected, and reduce probabilities which are higher than expected. Progressively, the network will reflect a situation corresponding to the real measurements provided in the training sample, such that a probability may be predicted when newly submitted sample are used as inputs. A symbolic representation in FIG. 3c illustrates ANN process 320, whereby various biological functions F are used as inputs 321 for cells or artificial neurons 322 including mathematical functions f, the neurons 322 having for example probabilities P of subsurface hydrocarbon productivity as outputs 323. Each mathematical function f comprising weights which are adjusted until the artificial neural system reflects a relationship between biological functions and probabilities close to the collected microbiome data.

Other techniques such as deep learning techniques may be used to relate biological functions of the accessed microbiome data with subsurface hydrocarbon productivity at the different geographical locations to identify biological function candidates indicative of subsurface hydrocarbon productivity.

Subsurface hydrocarbon productivity in this disclosure refers to the absence or presence of exploitable hydrocarbons in a specific location. The collected microbiome data can be classified in categories related to higher or lower subsurface hydrocarbon productivity. In an example, hydrocarbons include crude oil and natural gas.

The identified biological function candidates resulting from relating as per 202 biological functions of the accessed microbiome data with subsurface hydrocarbon productivity at the different geographical locations are biological functions which for example are present in geographical locations where subsurface hydrocarbon productivity is relatively high. On its own, such information is particularly precious due to the high costs of drilling wells to evaluate the presence or absence of hydrocarbons. Offering a computational alternative to drilling in order to identify areas of high potential for hydrocarbon extraction, or sweet spots, and that could be complementary to other standard exploration methods, is of great value.

In FIG 2, a probability profile of subsurface hydrocarbon productivity associated to the biological function candidates is established in 203. A probability profile may be represented as a probability as such, or may be represented by other means, including but not limited to maps or colors. A probability profile could for example imply that a high relative presence of a specific biological function or the presence of a group of functions fulfilling a criteria is highly indicative of subsurface hydrocarbon productivity. In an example, subsurface hydrocarbon productivity is highly probable in a geographical location if a sample in the geographical location comprises a group of specific biological functions within specific ranges. 203 can represent an instruction executable by a processor 101 encoded on a storage medium 102. 203 can also be comprised in a method according to the invention. In an example, the profile comprises a combination of biological function candidates and their related indication of subsurface hydrocarbon productivity. In an example, the microbiome data collected is based on data for which subsurface hydrocarbon productivity is known. In an example, the microbiome data collected includes data for which subsurface hydrocarbon productivity is known, and microbiome data for which subsurface hydrocarbon productivity is not known. In an example, the microbiome data for which the subsurface hydrocarbon productivity is known is used to train a model such as models 301, 310 or 320 for example, and the model is then used to classify microbiome data for which the subsurface hydrocarbon productivity is not known in order to predict a subsurface hydrocarbon productivity. A microbiome data collected at a geographical location comprises a number of associated biological functions representing its functional profile, such biological functions including biological function candidates if they are related to subsurface hydrocarbon productivity.

In FIG 2, biological function candidates which are not biological functions known to be indicative of subsurface hydrocarbon productivity are identified in 204. 204 can represent an instruction executable by a processor 101 encoded on a storage medium 102. 204 can also be comprised in a method according to the invention. Some biological functions are already known to be indicative of subsurface hydrocarbon productivity. Such biological functions include for example a hydrocarbon oxidizing function.

A hydrocarbon-oxidizing function is for example performed by bacteria, fungi or archaea that use volatile, gaseous hydrocarbon components, such as for example methane, ethane, propane and butane that migrate upward from subsurface hydrocarbon accumulations through natural microcracks in geologic structures (called microseepage), as a carbon source for their metabolic activities and growth. The processes for the oxidation of hydrocarbons comprise for example oxidation of methane producing methanol and forming formaldehyde under the presence of oxygen; oxidation of ethane, propane and n-butane producing alcohols, aldehyde and acetate, which can be assimilated into cell material.

Identifying biological function candidates which are not biological functions known to be indicative of subsurface hydrocarbon productivity can be extremely valuable in that such biological function candidates may permit detecting an exploiting subsurface hydrocarbons in locations in which these were not previously detected. Without wishing to be bound by theory, complex biological mechanisms may imply a relation between a biological function candidate and a strong indication of subsurface hydrocarbon productivity which was not previously detected. The present disclosure leverages the processing of data to detect such occurrences, thereby opening possibilities to detect and exploit locations which have not been detected or exploited yet.

In an example, a list of biological functions known to be indicative of subsurface hydrocarbon productivity is maintained. In an example, the identified biological function candidates which are not biological functions known to be indicative of subsurface hydrocarbon productivity are included in a list of biological functions known to be indicative of subsurface hydrocarbon productivity. In an example, models such as ANN, RF or SVM are trained iteratively using lists of biological functions known to be indicative of subsurface hydrocarbon productivity including biological function candidates identified in earlier iterations according to the disclosure. Such iterations enlarges the number of biological functions known to be indicative of subsurface hydrocarbon productivity, increasing the prediction capacity of machine learning algorithms, permitting in turn to better classify new samples due to the fact that their functional profile have been already been processed.

In an example, functional profiling data comprises functions obtained by a prediction. In an example, functional profiling data comprises metagenomics analysis. In an example, the functional profiling data comprises functions obtained by a prediction by sequencing a marker gene. In another example, the functional profiling data comprises functions obtained by a prediction by detecting or quantifying a marker gene in a soil sample. A preparation of the soil sample prior to the use of a detection or quantification technique can be performed, which can for example include nucleic acid extraction, nucleic acid fragmentation, cloning, elaboration of DNA libraries or protein libraries of the soil sample. Included among the examples of such techniques for the detection or quantification of a marker gene are for example sequencing, amplification or hybridization techniques, or a combination of these. Sequence of primers or probes can for example be derived from gene sequences as well as conditions which could allow gene detection or quantification, including temperature conditions, reaction times, reagents and buffers. Amplification techniques which can be used include: Polymerase Chain Reaction (PCR), real-time PCR (RT-PCR), quantitative real-time PCR (QPCR), multiplex PCR and Nucleic Acid Sequence Based Amplification (NASBA). In an example the technique used is sequencing. In another example the sequencing is ribonucleic acid (RNA) sequencing. In another example the sequencing is deoxyribonucleic acid (DNA) sequencing. Included among sequencing are the techniques such as, for example: Sanger sequencing, shotgun sequencing and high-throughput sequencing (Next-generation sequencing). Examples among the high-throughput sequencing can be the following: Ion semiconductor sequencing (Ion Torrent sequencing), sequencing by synthesis (Illumina sequencing), sequencing by ligation (SOLiD sequencing), Massively Parallel Signature sequencing (MPSS), pyrosequencing and DNA nanoball sequencing. In an embodiment of the present invention the sequencing is of genomic DNA or alternatively of plasmid DNA. In another embodiment the sequencing is of a marker gene.

Marker gene may refer to a gene that is involved in a biological function of the microorganism present in the soil sample; or, a gene that allows the distinction between genus or species of the microbiome present in a soil sample, for example that allows phylogenetic classification. The marker gene can for example be present in a plasmid or in a chromosomal segment.

A marker gene can be for example a small subunit ribosomal RNA (SSU rRNA) gene (16S or 18S), a large subunit (LSU) rRNA gene (23S or 28S), an Internal transcribed spacer (ITS) in the rRNA, or a chaperonin-60 (cpn60) gene. In an example the marker gene is the 16S rRNA gene. Regarding the 16S rRNA, one or more conserved, variable or hypervariable regions can be used for the detection or quantification, for example by sequencing, for example of variable regions such as V2, V4 or V6. The sequence and structure of 16S rRNA is for example disclosed in Yarza P. et al. "Uniting the classification of cultured and uncultured bacteria and archaea using 16S rRNA gene sequences". Nature Reviews Microbiology 2014 (12):635-645.

The preceding description has been presented to illustrate and describe certain examples. Different sets of examples have been described; these may be applied individually or in combination, sometimes with a synergetic effect. This description is not intended to be exhaustive or to limit these principles to any precise form disclosed. Many modifications and variations are possible in light of the above teaching. It is to be understood that any feature described in relation to any one example may be used alone, or in combination with other features described, and may also be used in combination with any features of any other of the examples, or any combination of any other of the examples.

## Claims

1. A non-transitory machine-readable storage medium encoded with instructions executable by a processor, the machine-readable storage medium comprising:
instructions to access microbiome data collected at different geographical locations, the microbiome data comprising functional profiling data;
instructions to relate biological functions of the accessed microbiome data with subsurface hydrocarbon productivity at the different geographical locations to identify biological function candidates indicative of subsurface hydrocarbon productivity;
instructions to establish a probability profile of subsurface hydrocarbon productivity associated to the biological function candidates;
instructions to identify biological function candidates which are not biological functions known to be indicative of subsurface hydrocarbon productivity.

2. A non-transitory machine-readable storage medium according to claim 1, whereby the instructions to relate biological functions of the accessed microbiome data with subsurface hydrocarbon productivity at the different geographical locations to identify biological function candidates indicative of subsurface hydrocarbon productivity comprise using a machine learning algorithm.

3. A non-transitory machine-readable storage medium according to any of the above claims, whereby the functional profiling data comprises functions obtained by a prediction by sequencing a marker gene.

4. A non-transitory machine-readable storage medium according to any of the above claims, whereby the identified biological function candidates which are not biological functions known to be indicative of subsurface hydrocarbon productivity are included in a list of biological functions known to be indicative of subsurface hydrocarbon productivity.

5. A non-transitory machine-readable storage medium according to any of the above claims, whereby the biological functions known to be indicative of subsurface hydrocarbon productivity comprise a hydrocarbon oxidizing function.

6. A computer system comprising a processor, a storage medium coupled to the processor, and an instruction set to cooperate with the processor and the storage medium to:
access microbiome data collected at different geographical locations, the microbiome data comprising functional profiling data;
relate biological functions of the accessed microbiome data with subsurface hydrocarbon productivity at the different geographical locations to identify biological function candidates indicative of subsurface hydrocarbon productivity;
establish a probability profile of subsurface hydrocarbon productivity associated to the biological function candidates;
identify biological function candidates which are not biological functions known to be indicative of subsurface hydrocarbon productivity.

7. A computer system according to claim 6, whereby the instruction set is to cooperate with the processor and the storage medium to relate biological functions of the accessed microbiome data with subsurface hydrocarbon productivity at the different geographical locations to identify biological function candidates indicative of subsurface hydrocarbon productivity comprising using a machine learning algorithm.

8. A computer system according to any claims 6 or 7, whereby the functional profiling data comprises functions obtained by a prediction by sequencing a marker gene.

9. A computer system according to any of claims 6 to 8, whereby the identified biological function candidates which are not biological functions known to be indicative of subsurface hydrocarbon productivity are included in a list of biological functions known to be indicative of subsurface hydrocarbon productivity.

10. A computer system according to any of the claims 6 to 9, whereby the biological functions known to be indicative of subsurface hydrocarbon productivity comprise a hydrocarbon oxidizing function.

11. A method to identify biological functions comprising:
accessing microbiome data collected at different geographical locations, the microbiome data comprising functional profiling data;
relating biological functions of the accessed microbiome data with subsurface hydrocarbon productivity at the different geographical locations to identify biological function candidates indicative of subsurface hydrocarbon productivity;
establishing a probability profile of subsurface hydrocarbon productivity associated to the biological function candidates;
identifying biological function candidates which are not biological functions known to be indicative of subsurface hydrocarbon productivity.

12. A method according to claim 11, whereby relating biological functions of the accessed microbiome data with subsurface hydrocarbon productivity at the different geographical locations to identify biological function candidates indicative of subsurface hydrocarbon productivity comprise using a machine learning algorithm.

13. A method according to any of claims 11 or 12, whereby the functional profiling data comprises functions obtained by a prediction by sequencing a marker gene.

14. A method according to any of claims 11 to 13, whereby the identified biological function candidates which are not biological functions known to be indicative of subsurface hydrocarbon productivity are included in a list of biological functions known to be indicative of subsurface hydrocarbon productivity.

15. A method according to any of claims 11 to 14, whereby the biological functions known to be indicative of subsurface hydrocarbon productivity comprise a hydrocarbon oxidizing function.
